# EUROPÄISCHE PATENTSCHRIFT

(11) **EP 0 718 408 B1**
(45) Veröffentlichungstag und Bekanntmachung des Hinweises auf die Patenterteilung: **13.08.2003**
(21) Anmeldenummer: 95118269.0
(22) Anmeldetag: 21.11.1995
(51) Int. Cl.: C12Q 1/68

(54) **Verfahren zum besonders sensitiven Nachweis von Nukleinsäuren**
Sensitive method for detecting nucleic acids
Méthode sensible pour détecter des acides nucléiques

(30) Priorität: 23.11.1994 DE 4441626
(43) Veröffentlichungstag der Anmeldung: 26.06.1996
(73) Patentinhaber: Roche Diagnostics GmbH, 68298 Mannheim (DE)
(72) Erfinder: Seibl, Rudolf, Dr., D-82377 Penzberg (DE); Rosemeyer, Viola, B-1300 Wavre (BE)

(56) Entgegenhaltungen:
- WO-A-93/19202
- DE-A- 4 431 269
- CLONEY ET AL: "A SIMPLE, HIGHLY SENSITIVE, NUCLEIC ACID AMPLIFICATION SYSTEM" CLINICAL CHEMISTRY, Bd. 40, Nr. 4, 1.Januar 1994, Seite 656 XP002042545

## Beschreibung

Gegenstand der Erfindung ist ein Verfahren zum besonders sensitiven Nachweis von Nukleinsäuren durch Hybridisierung einer Sondennukleinsäure mit einer Zielnukleinsäure, Abbau des hybridisierten Teils der Sondennukleinsäure und Nachweis des Spaltprodukts sowie ein hierfür geeignetes Set von Reagenzien.

Nachdem sich herausgestellt hatte, daß die Ausnutzung der spezifischen Information von Nukleinsäuren große Vorteile bei der Erkennung von Infektionsparametern und genetischen Zuständen haben könnte, wurde versucht, Nukleinsäuren zum Gegenstand von Testverfahren zu machen. In vielen Fällen ist für einen Nachweis aber die Vermehrung der Nukleinsäuresequenzen erforderlich, um überhaupt ein ausreichendes Signal in dem verwendeten Nachweisverfahren erzeugen zu können. Dafür wurden sowohl Verfahren zur Vermehrung einer Zielsequenz als auch Verfahren unter Vermehrung einer zielsequenzunabhängigen Nukleinsäuresequenz vorgeschlagen. Ein Beispiel für eine Vermehrung der Zielsequenz ist die in EP-A-0 201 184 beschriebene Polymerasekettenreaktion (PCR), in der die beiden Stränge der Zielnukleinsäure in einer in-vitro-Replikationsreaktion amplifiziert werden. Die Temperatur der Reaktion wird zyklisch verändert, um eine doppelsträngige Zielnukleinsäure zu denaturieren, die Hybridisierung mit Initiatormolekülen (Primern) und die anschließende DNA-Synthese (Verlängerung der Primer) zu ermöglichen und diese Schritte zu wiederholen. Sofern alle Schritte der PCR optimal laufen, ergibt sich eine exponentielle Amplifikation von Nukleinsäuren. In der Praxis ist der Amplifikationsfaktor jedoch oftmals stark verringert.

Für die Vermehrung von Zielsequenzen wurden auch in-vitro-Transkriptionen verwendet. Ein Beispiel für eine solche Reaktion ist in WO 88/10315 beschrieben. Bei dieser Amplifikation werden 2 Primer eingesetzt, von denen einer eine Promotorsequenz enthält. Die Primer sind wie bei der PCR zu unterschiedlichen Strängen der zu amplifizierenden Sequenz komplementär. In EP-A-0 329 822 ist eine Weiterentwicklung dieses Verfahrens beschrieben, bei der die durch Transkription des als Zwischenprodukt entstandenen doppelsträngigen Nukleinsäureprodukts entstandenen Ribonukleinsäuren im Hybrid abgebaut werden, um eine Hybridisierung der neu synthetisierten DNA mit dem promotorhaltigen Primermolekül zu ermöglichen. Durch den Abbau der ursprünglich gebildeten Transkripte wird es möglich, aus jedem gebildeten RNA-Molekül erneut eine doppelsträngige promotorhaltige Nukleinsäure zu bilden, die für einen erneuten Transkriptionsstart verwendet werden kann.

Im Gegensatz zu den vorangehend geschilderten Methoden dient die zielsequenzspezifische Signalverstärkung nicht zur Vermehrung der nachzuweisenden Nukleinsäure, sondern hat nur die spezifische Identifizierung der Zielsequenz zum Ziel. Um dies zu erreichen, wird ein Signal in Abhängigkeit von der Anwesenheit der Zielsequenz verstärkt. Eine solche Methode ist z. B. in WO 89/09284 und WO 89/10415 beschrieben. In dieser sogenannten Cycling-Probe-Reaktion (CPR) wird ein markiertes, chimäres DNA-RNA-DNA-Sondenmolekül eingesetzt, das mit einem Ziel-DNA-Molekül hybridisiert. Durch die Hybridisierung entsteht ein RNA-DNA-Hybrid, das ein Subtrat für die RNAse H darstellt. Da dieses Enzym spezifisch den RNA-Anteil des Hybrids abbaut, wird das Sondenmolekül gespalten und die entstehenden Fragmente diffundieren aufgrund der geringeren Schmelztemperatur von der Zielsequenz ab. Daraufhin kann das Zielmolekül mit einem weiteren Sondenmolekül hybridisieren und der Zyklus wird wiederholt. Die Fragmente des Sondenmoleküls werden über die daran befindliche Markierung nachgewiesen.

Ziel der vorliegenden Erfindung war es, die Empfindlichkeit von Verfahren zu erhöhen, bei denen eine Zielnukleinsäure mit einem Sondenmolekül hybridisiert wird und der Abbau des Sondenmoleküls zur Bestimmung der Zielnukleinsäure verwendet wird.

Gegenstand der Erfindung ist ein Verfahren zum Nachweis von Ziel-Nukleinsäuren, enthaltend die Schritte
a) zielsequenzabhängige Erzeugung einer Vielzahl von Nukleinsäuren, welche als Primer an einer Matrizennukleinsäure fungieren können, aus Nukleinsäuren mit einer längeren Sequenz,
b) Amplifikation der Nukleinsäuren, welche als Primer an einer Matrizennukleinsäure fungieren können, oder von Teilen davon,
c) Nachweis der Zielnukleinsäure aufgrund eines der in der Amplifikation gebildeten Produkte oder Zwischenprodukte.

Ebenfalls Gegenstand der Erfindung ist ein Verfahren zur Erzeugung von Nukleinsäuresequenzen mit Hilfe von längeren Nukleinsäuresequenzen, die die kurzen Nukleinsäuresequenzen enthalten, ein Verfahren zum empfindlichen Nachweis einer Zielnukleinsäure A und Kits zur Durchführung dieser Verfahren.

Unter Zielnukleinsäuren werden Nukleinsäuren verstanden, die entweder direkt oder indirekt nachgewiesen werden sollen. Im folgenden und insbesondere in den Zeichnungen wird die Zielnukleinsäure mit dem Buchstaben A gekennzeichnet. Unter Verfahren zum direkten Nachweis von Zielnukleinsäuren werden solche verstanden, bei denen die Nukleinsäuren bereits für die erfindungsgemäßen Verfahrensschritte bereitstehen. Unter indirekten Verfahren werden solche verstanden, bei denen die Zielnukleinsäuren einem Vorbehandlungsschritt unterzogen bzw. durch diese bereitgestellt werden. Solche Vorbehandlungsschritte können beispielsweise die Isolierung der Nukleinsäuren, die Behandlung mit Reagenzien, z. B. Restriktionsenzymen, oder die Voramplifikation darstellen. Auch die reverse Transkription von RNA kann als Vorbehandlung angesehen werden. Die Zielnukleinsäure kann daher beliebigen, beispielsweise viralen, bakteriellen oder zellulären Ursprungs sein. Sie kann in Lösung, Suspension, aber auch an Festkörpern fixiert oder in zellhaltigen Medien, Zellabstrichen, fixierten Zellen, Gewebsschnitten oder fixierten Organismen vorliegen. Bevorzugt liegt die Nukleinsäure in Lösung vor.

Unter komplementären bzw. homologen Nukleinsäuren werden Nukleinsäuren verstanden, die mit der entsprechenden Nukleinsäure hybridisieren können bzw. mit einer Nukleinsäure hybridisieren können, die komplementär zu der entsprechenden Nukleinsäure ist.

Üblicherweise beginnen die Nachweisverfahren für Nukleinsäuren mit einem Schritt zur Verfügbarmachung der Zielnukleinsäure mit entsprechenden Reagenzien. Hierbei können sowohl Veränderungen des pHs (alkalisch), Hitze, Wiederholung extremer Temperaturveränderungen (Einfrieren/Auftauen), Veränderung der physiologischen Wachstumsbedingungen (osmotischer Druck), Einwirkung von Detergentien, chaotropen Salzen oder Enzymen (z. B. Proteasen, Lipasen) allein oder in Kombination zur Freisetzung der Nukleinsäuren beitragen. Da das erfindungsgemäße Verfahren sehr empfindlich und selektiv ist, können auch kleine Nukleinsäuremengen in Anwesenheit anderer Stoffe, beispielsweise Proteinen, Zellen, Zellfragmenten, aber auch nicht nachzuweisender Nukleinsäuren nachgewiesen werden. Geeignete Zielnukleinsäuren sind beispielsweise Ribonukleinsäuren und Deoxyribonukleinsäuren. Die Nukleinsäuren können auch modifiziert sein, beispielsweise durch die vorher vorgenommenen Bearbeitungsschritte. Besonders bevorzugt als Zielnukleinsäure ist Deoxyribonukleinsäure (DNA).

Bei dem erfindungsgemäßen Verfahren handelt es sich um eine spezielle Ausführungsform von auf einem Hybridisierungsereignis beruhenden Test, im speziellen der zielsequenzspezifischen Signalverstärkung. Auf Hybridisierungsereignisse beruhende Tests sind in ihren Grundzügen dem Fachmann auf dem Gebiet der Nukleinsäurediagnostik bekannt. Soweit experimentelle Details im folgenden nicht ausgeführt sind, wird dazu vollinhaltlich auf "Nucleic acid hybridisation", Herausgeber B.D. Hames und S.J. Higgins, IRL Press, 1986, z. B. in den Kapiteln 1 (Hybridisation Strategy), 3 (Quantitative Analysis of Solution Hybridisation) und 4 (Quantitative Filter Hybridisation), Current Protocols in Molecular Biology, Edt. F.M. Ausubel et al., J. Wiley and Son, 1987, und Molecular Cloning, Edt. J. Sambrook et al., CSH, 1989, Bezug genommen. Zu den bekannten Methoden gehört auch die Herstellung von markierten Nukleosidtriphosphaten, wie sie in EP-A-0 324 474 beschrieben sind, die chemische Synthese von modifizierten und unmodifizierten Oligonukleotiden, die Spaltung von Nukleinsäuren mittels Restriktionsenzymen, die Auswahl von Hybridisierungsbedingungen, durch welche eine Spezifität erreicht werden kann, die unter anderem vom Ausmaß der Komplementarität zwischen den zu hybridisierenden Nukleinsäuren, deren GC-Gehalt und deren Länge abhängt, sowie die Bildung von Nukleinsäuren aus Nukleosidtriphosphaten mit Hilfe von Polymerasen, gegebenenfalls unter Verwendung von sogenannten Primern.

Eine Markierung im Sinne der vorliegenden Erfindung besteht aus einer direkt oder indirekt nachweisbaren Gruppe. Direkt nachweisbare Gruppen sind beispielsweise radioaktive (³²P), farbige, oder fluoreszierende Gruppen oder Metallatome. Indirekt nachweisbare Gruppen sind beispielsweise immunologisch oder enzymatisch wirksame Verbindungen, wie Antikörper, Antigene, Haptene oder Enzyme oder enzymatisch aktive Teilenzyme. Diese werden in einer nachfolgenden Reaktion oder Reaktionssequenz detektiert. Besonders bevorzugt sind Haptene, da mit ihnen markierte Nukleosidtriphosphate im allgemeinen besonders gut als Substrate von Polymerasen einsetzbar sind und eine anschließende Reaktion mit einem markierten Antikörper gegen das Hapten oder das haptenisierte Nukleosid leicht vorgenommen werden kann. Solche Nukleosidtriphosphate sind beispielsweise Brom-Nukleosidtriphosphate oder Digoxigenin-, Digoxin-, Biotin- oder Fluorescein-gekoppelte Nukleosidtriphosphate. Als besonders geeignet haben sich die in EP-A-0 324 474 genannten Steroide und deren Detektion erwiesen. Für deren Inkorporation in Nukleinsäuren wird hiermit auf die EP-A-0 324 474 verwiesen.

Nukleosidtriphosphate (NTP) sind Ribo (rNTP)- oder Desoxyribo-Nukleosidtriphosphate (dNTP).

Charakteristisch für das vorliegende Verfahren ist, daß mit Hilfe und in Abhängigkeit von der Anwesenheit der Zielnukleinsäuren aus Nukleinsäuren mit einer längeren Sequenz eine Vielzahl von Nukleinsäuren mit einer kurzen Sequenz erzeugt werden, welche als Primer fungieren können. Als längere Sequenz wird eine Sequenz bezeichnet, die um 1 oder mehr, bevorzugt 5 - 1000, besonders bevorzugt 8 - 100, Nukleobaseneinheiten (z. B. Mononukleotide) länger ist als die kurze Nukleinsäure. An die kurze Nukleinsäure werden zwei Bedingungen geknüpft. Zunächst muß sie mit Nukleinsäuren über Base-Base-Wechselwirkungen Hybride bilden können. Solche Sequenzen enthalten insbesondere Nukleinsäuren, die aus den natürlichen Nukleotidbausteinen aufgebaut sind. Bei den Sequenzen kann es sich jedoch beispielsweise auch um Nukleinsäureanaloge oder modifizierte Nukleinsäuren, z. B. Nukleinsäuren handeln, die zwar noch die Hybridisierungseigenschaften einer Nukleinsäure aufweisen, jedoch keinen Phosphat-Zucker-Kettenanteil haben, oder aber nicht-natürliche Basen aufweisen. Hierfür sind insbesondere Moleküle der WO 92/20702 oder WO 86/05518 geeignet. Die zweite Bedingung für die Sequenz ist, daß sie als Primer als Verlängerungsreaktion verwendet werden kann. Solche Verlängerungsreaktionen sind insbesondere die Polymerase-abhängige Verlängerung der Primer um Mononukleotideinheiten unter Verwendung von Mononukleosidtriphosphaten. Hierbei wird das 3'-Ende des Primers im Zustand der Hybridisierung mit einer Matrizennukleinsäure um Mononukleotide verlängert, so daß das Verlängerungsprodukt im wesentlichen komplementär zu der korrespondierenden Stelle auf der Matrizennukleinsäure ist.

Die erfindungsgemäße Erzeugung der Primer bzw. kurzen Nukleinsäure kann auf beliebige Weise geschehen, bevorzugt ist jedoch die Spaltung einer längeren Nukleinsäure, insbesondere durch partiellen enzymatischen Abbau dieser Nukleinsäure.

Die im erfindungsgemäßen Schritt erzeugten Primer haben bevorzugt eine Länge von mehr als 12 Basen. Die Primer enthalten bevorzugt eine Sequenz von 12 bis 35 Basen, welche zum Weiterbearbeiten der Primer (z. B. Hybridisierung und enzymatische Verlängerung) unerläßlich ist. Die Primer können, insbesondere an ihrem 5'-Ende, noch gewünscht viele weitere Nukleotide oder nicht-nukleotidische Molekülteile enthalten.

Im zweiten Schritt des erfindungsgemäßen Verfahrens werden die im ersten Schritt gebildeten Primer oder Teile davon, z. B. eine Teilsequenz, amplifiziert. Bevorzugt schließt auch die Amplifikation des Primers einen Abbau eines längeren Moleküls ein. Besonders bevorzugt jedoch geschieht die Amplifikation durch Verlängerung, z. B. eine längere Sequenz verglichen mit dem Primer (kurze Sequenz), des Primers an einer Matrizennukleinsäure C (zu einer längeren Nukleinsäure), Hybridisierung einer Amplifikatorsonde D mit dem Verlängerungsprodukt des Primers und Abbau (Spaltung) der Amplifikatorsonde unter Erzeugung eines neuen Primermoleküls. Dadurch, daß das Verlängerungsprodukt dieses Primers erneut zur Spaltung einer Amplifikatorsonde zur Verfügung steht, wächst die Anzahl der Primermoleküle mit jedem Spaltvorfall. Darüber hinaus führt die Verlängerung der durch die Spaltung gebildeten Primer erneut zur Bildung von Verlängerungsprodukten, die wieder zur Spaltung einer Amplifikatorsonde zur Verfügung stehen.

Dadurch, daß die erstmalige Erzeugung eines Primers zielsequenzabhängig ist, ist die Menge an erzeugten Primern bzw. Verlängerungsprodukten oder weiteren Zwischenprodukten ein Maß für die Anwesenheit von Zielnukleinsäuren A in einer Probe.

Bei dem erfindungsgemäßen Verfahren handelt es sich daher um ein Verfahren, bei dem sowohl ein einmal erzeugtes Verlängerungsprodukt eines Primers wieder zur zyklischen Erzeugung eines oder mehrerer Primer benutzt wird, als auch ein einmal erzeugter Primer wieder zur Erzeugung eines Verlängerungsproduktes benutzt wird.

Unter einer längeren Nukleinsäure wird hier eine Nukleinsäure verstanden, die einen inaktiven Primierteil und einen Teil enthält, der, wenn er abgebaut wird, zu einer Aktivierung des Primers führt. Nach Aktivierung des Primers steht dieser zur Durchführung einer Verlängerungsreaktion zur Verfügung. Ein Aspekt der Erfindung befaßt sich mit der Amplifikation von kurzen Nukleinsäuren, insbesondere Primern, durch Abbau der längeren Nukleinsäuren unter Bildung der Primer. Im Sinne der vorliegenden Erfindung sind auch die Sondennukleinsäuren B solche längeren Nukleinsäuren.

Kurze Nukleinsäuren im Sinne der Erfindung sind die Abbauprodukte der oben genannten längeren Nukleinsäuren. Insbesondere sind die kurzen Nukleinsäuren Nukleinsäuren, die als Primer fungieren können, d. h. um einen Verlängerungsbereich verlängert werden können.

Als Sondennukleinsäuren B sind Moleküle geeignet, die zwei miteinander verbundene Teile B1 und B2 enthalten. Teil B ist dadurch charakterisiert, daß er mit der Zielnukleinsäure bzw. einem Teil davon hybridisieren kann. Dazu ist dieser Teil ausreichend komplementär. Darüberhinaus muß der Teil B1 gespalten werden können, wenn er in mit der Zielnukleinsäure hybridisierter Form vorliegt. Unter Abbau oder Spaltung ist hierbei die Aufteilung des Teiles B1 in zwei oder mehr nicht mehr aneinander gebundene Teile oder Abspaltung des Teils B1 von B2 zu verstehen, wobei die Zielnukleinsäure bevorzugt nicht gespalten wird. Teil B1 kann daher z. B. ribonukleotidische oder abasische Sequenzen enthalten. In einem bevorzugten Fall enthält Teil B1 zwei, besonders bevorzugt vier oder mehr über die in Nukleinsäuren übliche Weise miteinander verknüpfte Monoribonukleotideinheiten, während der hierzu komplementäre Teil der Zielnukleinsäure eine Deoxyribonukleinsäure darstellt. In diesem Fall kann eine Spaltung der Sondennukleinsäure B im Teil B1 dadurch geschehen, daß das gebildete Hybrid mit RNAse H in Kontakt gebracht wird. Im Falle der Anwesenheit abasischer Sequenzen kann der Abbau mittels AP-Endonuklease geschehen.

Bei ausreichender Empfindlichkeit kann eventuell eine Spaltung auch der Zielnukleinsäure in Kauf genommen werden, d. h. für jedes Zielmolekül wird ein Primer erzeugt anstelle von mehreren.

Dabei wird mindestens ein Teil des hybridisierbaren Teiles B1 gespalten. Dadurch bildet sich ein Spaltprodukt B', welches den im Hybrid von B mit der Zielnukleinsäure nicht spaltbaren und bevorzugt nicht mit der Zielnukleinsäure hybridisierenden nukleinsäurespezifischen Teil B2, sowie eventuell Teile des ursprünglich mit der Zielnukleinsäure hybridisierten Teiles B1 enthält.

Die Bedingungen für die Hybridisierung der Zielnukleinsäure mit der Sondennukleinsäure werden vorzugsweise so gewählt, daß noch eine selektive Hybridisierung der Sondennukleinsäure über Teil B1 mit der Zielnukleinsäure stattfindet, eine unselektive Hybridisierung der Sondennukleinsäure mit anderen, nicht nachzuweisenden Nukleinsäuren der Probe aber nicht mehr stattfindet. Die durch die Spaltung der Sondennukleinsäure entstehenden Bruchstücke, darunter auch das Spaltprodukt B', sind kürzer als die ursprüngliche Sondennukleinsäure und werden unter den gewählten Bedingungen daher kein stabiles Hybrid mehr mit der Zielnukleinsäure bilden können. Unter den gewählten Bedingungen werden sie daher die Zielnukleinsäure A freigeben. Diese steht für eine Hybridisierung mit einer weiteren Sondennukleinsäure bereit.

Durch Spaltung der Sondennukleinsäure können auch unterschiedliche Bruchstücke B' entstehen, welche entweder nur den Teil B2 alleine enthalten oder aber zusätzlich noch Reste des Teils B1. Dies hängt von den jeweils verwendeten Bedingungen ab.

Unter einem nukleinsäurespezifischen Teil einer Nukleinsäure wird im Folgenden eine Sequenz verstanden, die mit einer anderen Sequenz als der Zielnukleinsäure hybridisieren kann, wobei unter den gewählten Bedingungen eine spezifische Hybridisierung stattfindet, d. h. keine für das Gesamtverfahren relevante Hybridisierung mit anderen an dem jeweiligen Reaktionsschritt unbeteiligten in der Reaktionsmischung vorhandenen Nukleinsäuren stattfindet. Typische nukleinsäurespezifische Teile sind Teil B2 der Sondennukleinsäure, Teil C2 der Matrizennukleinsäure und Teil D2 der Amplifikatorsonde.

Der bevorzugt nicht mit der Zielnukleinsäure hybridisierende, Nukleinsäure-spezifische Teil B2 der Sondennukleinsäure muß die Bedingung erfüllen, daß er unter den Bedingungen des zielsequenzspezifischen Abbaus des Teiles B1 nicht abgebaut wird. Außerdem soll er mit dem später definierten Matrizenoligonukleotid hybridisieren können. Die Sequenz des Teils B2 kann prinzipiell frei gewählt werden. Hierbei ist jedoch zu berücksichtigen, daß die Hybridisierung des Spaltprodukts B' mit dem Matrizenoligonukleotid durch eine Komplementarität mit der Zielnukleinsäure erschwert wird, wodurch voraussichtlich die Sensitivität gegenüber dem optimalen Fall erniedrigt ist. B2 kann ebenfalls eine Ribonukleinsäure, Deoxyribonukleinsäure oder modifizierte Nukleinsäure sein. Für den Fall, daß der Teil B eine Ribonukleinsäure enthält und die Spaltung mittels einer RNAse vorgenommen wird, stellt Teil B2 bevorzugt keine unter diesen Bedingungen spaltbare Ribonukleinsäure dar, bevorzugt stellt er Deoxyribonukleinsäure dar. B2 kann jedoch auch eine so modifizierte Ribonukleinsäure sein, daß sie nicht mehr von einer RNAse gespalten werden kann. Eine weitere Möglichkeit ist die Verwendung von Nukleinsäureanalogen, die zwar noch die Hybridisierungseigenschaften einer Nukleinsäure aufweisen, jedoch kein Phosphat-Zucker-Kettenanteil oder nicht-natürliche Basen haben. Hierfür sind insbesondere PNA-Moleküle der WO 92/20702 oder Moleküle der WO 86/05518 geeignet. Die bevorzugte Bedingung, daß dieser Anteil nicht mit der Zielnukleinsäure hybridisieren soll, bezieht sich insbesondere auf die während der Hybridisierung der Sondennukleinsäure mit der Zielnukleinsäure angelegten Bedingungen. Bevorzugt wird der Teil B2 so gewählt, daß er auch nicht mit nicht nachzuweisenden Nukleinsäuren der Probe hybridisiert. Teil B2 soll jedoch eine Sequenz beinhalten, die mit einer Matrizennukleinsäure C hybridisieren kann. Die Bedingungen der Hybridisierungen der Zielnukleinsäure mit der Sondennukleinsäure und der Matrizennukleinsäure mit dem Spaltprodukt B' können unabhängig gewählt werden.

Die erfindungsgemäße Matrizennukleinsäure C enthält einen Teil C2, der mit dem Spaltprodukt B' und insbesondere dessen nukleinsäurespezifischen Teil B2 oder einem Teil davon und gegebenenfalls eventuell noch vorhandenen Resten von B 1 hybridisieren kann. Als Matrizennukleinsäure C sind alle Moleküle geeignet, die eine solche Hybridisierung erlauben, d. h. Nukleinsäuren, die aus den natürlichen Nukleotidbausteinen aufgebaut sind, jedoch auch Nukleinsäureanaloge, die aus nicht natürlich vorkommenden Bausteinen aufgebaut sind oder solche Bausteine enthalten und als Template/Matrize fungieren können. Darüberhinaus sollte die Matrizennukleinsäure stabil gegenüber Abbau unter den gewählten Bedingungen sein. Für den Fall, daß die Spaltung der Sondennukleinsäure mit RNAse vorgenommen wird, handelt es sich bei der Matrizennukleinsäure besonders bevorzugt um Deoxyribonukleinsäure. Neben dem Teil C2 weist die Matrizennukleinsäure einen Teil C1 auf, der nicht mit dem Teil B1 bzw. noch vorhandenen Resten des Teils B 1 der Sondennukleinsäure hybridisieren kann. Besonders bevorzugt bildet dieser Teil C1 auch mit nicht nachzuweisenden Nukleinsäuren in der Probe und der Zielnukleinsäure selbst keine unter den gewählten Bedingungen stabilen Hybride aus. Bevorzugt liegt der Teil C1 in 5'-Richtung, gesehen vom Teil C2.

In jedem Fall sollte gewährleistet sein, daß das oder die Spaltprodukte B' mit der Matrizennukleinsäure derartig hybridisieren können, daß B' auch an dem durch Spaltung entstandenen Ende mit der Matrizennukleinsäure hybridisiert.

In einem anschließenden Schritt wird das Hybrid aus Spaltprodukt B' und Matrizennukleinsäure C um einen zu Teil C1 komplementären Nukleinsäureteil B3 verlängert. Daher muß B' als Primer dienen können, d. h. muß am 3'-Ende an der Matrize verlängert werden können. Dies kann auf beliebige Weise, bevorzugt jedoch durch enzymatische Verlängerung, geschehen. Als enzymatische Verlängerung wird besonders bevorzugt die Ankondensation von Mononukleosidtriphosphaten unter Verwendung von C1 als Templatnukleinsäure verstanden. Die angewandten Bedingungen sind dem Fachmann bekannt. Zum Beispiel kann die Verlängerung mit Hilfe von DNA-Polymerase aus Thermus aquaticus gemäß EP-A-0 258 018 durchgeführt werden. Als bevorzugte Mononukleosidtriphosphate sind dNTPs anzusehen. Für diesen Fall wirkt das Spaltprodukt B' als Primer und kurze Nukleinsäure, die verlängert wird. Eine weitere Möglichkeit der enzymatischen Verlängerung ist die Verwendung einer Ligase. Es sollte darauf geachtet werden, daß das gebildete Verlängerungsprodukt unter den Bedingungen der Spaltung der Sondennukleinsäure stabil ist Dies ist beispielsweise erfüllt, wenn es sich sowohl beim Primer als auch bei dem Verlängerungsstück um DNA handelt, wenn als Mittel zur Spaltung der Sondennukleinsäure RNAse H eingesetzt wird.

Unter den Bedingungen der Verlängerung des Spaltprodukts B' findet eine Verlängerung eventuell gebildeter Hybride aus ungespaltener Sondennukleinsäure B und Matrizennukleinsäure C nicht statt, da z. B. das 3'-Ende der Sondennukleinsäure nicht mit der Matrizennukleinsäure hybridisiert.

Die Verlängerung von ungespaltenen Sondennukleinsäuren B an der Zielnukleinsäure als Matrize beeinflußt die Erzeugung der Primer B' nicht und kann durch Blockierung des 3'-Endes der Sondennukleinsäure B beispielsweise durch Verwendung eines Didesoxynukleotides als letztes Nukleotid verhindert werden.

In einer Ausführungsform des erfindungsgemäßen Verfahrens wird die Amplifikation der Primer dadurch realisiert, daß bevorzugt nach Denaturierung des Hybrids aus Matrizennukleinsäure und des um B3 verlängerten Primers B' die Bildung eines Bereiches B3 zur Hybridisierung von B3 mit einer Amplifikatorsonde D verwendet wird.

Unter einer Amplifikatorsonde D wird eine Nukleinsäure verstanden, welche einen Teil D1 enthält, der auf seine gesamte Länge oder teilweise komplementär zu B3 ist, also dem durch Verlängerung entstandenen Teil des Verlängerungsproduktes des Primers (Spaltproduktes B'). Der neu an den Primer angehängte Teil wird im folgenden mit B3 bezeichnet. Ein wesentliches Merkmal der Amplifikatorsonde D ist, daß sie in ihrem Teil D1 wiederum spaltbar ist. Bevorzugt ist sie so gestaltet, daß die Spaltbedingungen für die Amplifikatorsonde und die Sondennukleinsäure B im wesentlichen identisch sind im Hinblick auf die eingesetzten Reagenzien. Daher ist D1 ebenfalls bevorzugt eine Ribonukleinsäure. Außerdem enthält die Amplifikatorsonde D einen Teil D2, der die Bedingungen einer nukleinsäurespezifischen Sequenz erfüllt.

Der besondere Amplifikationseffekt des Verfahrens ergibt sich dadurch, daß durch den Abbau der Amplifikatorsonde direkt oder indirekt wieder ein Spaltprodukt geschaffen wird, das mit einer Matrizennukleinsäure hybridisieren und somit (als Primer) einen erneuten Amplifikationszyklus einleiten kann.

Zusätzlich können bei geeigneten Bedingungen pro Verlängerungsprodukt mehrere Amplifikationssonden nacheinander binden, so daß mehrere Spaltprodukte entstehen, da analog zur Sondennukleinsäure B die Spaltprodukte kein stabiles Hybrid mit dem Verlängerungsprodukt bilden.

Bei der direkten Schaffung wird die Amplifikatorsonde so gewählt, daß der weitere Teil D2 auf die volle Länge oder nur in Teilen komplementär zu C2 und somit im wesentlichen identisch mit den ursprünglich gebildeten Spaltprodukten B' ist. Somit können die in Abbaureaktion von D gebildeten Spaltprodukte D' erneut in die Verlängerungsreaktion unter Verwendung der Matrizennukleinsäure C als Templat eingesetzt werden.

Bezüglich der Art und Anordnung der Teile D1 und D2 gelten für die Amplifikatorsonden die Bedingungen für die Sondennukleinsäure B.

In einem indirekten Verfahren wird die Amplifikatorsonde D so gewählt, daß sie außer dem Teil D1 einen nukleinsäurespezifischen Teil D2 enthält, der jedoch nicht komplementär zu C2 ist und auch mit weiteren Nukleinsäuren, die in der Reaktionsmischung enthalten sind, nicht hybridisiert, mit Ausnahme einer weiteren Matrizennukleinsäure E, die einen mit D2 hybridisierbaren Teil E2 und einen als Templat zur Verlängerung von D' wirkenden Teil E1 aufweisen. Nach Hybridisierung des Spaltstückes D' mit der zweiten Matrizennukleinsäure E und Verlängerung des Spaltstückes und Primers um einen Teil D3 steht der neu gebildete Teil D3 analog dem Teil B3, bevorzugt nach Hybridtrennung, zur Spaltung einer weiteren Amplifikatorsonde F bereit.

Diese Amplifikatorsonde F kann wiederum einen zu D3 komplementären Teil F1 und einen nukleinsäurespezifischen Teil F2 enthalten. Nach Spaltung im Teil F1 entsteht ein Spaltstück F, welches je nach Wahl der Sequenz gleichwirkend ist mit Spaltprodukt D' oder Spaltprodukt B'. Ein noch weiter erhöhter Amplifikationsgrad wird erreicht, wenn das Spaltstück wieder in einen Verlängerungs-/Abbauzyklus unter Verwendung weiterer Matrizen- und Amplifikatorsonden eingeführt wird. Das erfindungsgemäße System ist daher durch die Wahl der Sequenzen des nukleinsäurespezifischen Teils in der Sondennukleinsäure bzw. der Amplifikatorsonden sehr flexibel. Da zu erwarten ist, daß bei zunehmender Anzahl von eingesetzten Sonden- und Matrizennukleinsäuren eine große Vielfalt von diversen Molekülen entsteht, ist die Komplexität mit zunehmender Menge von unterschiedlichen Sonden- bzw. Matrizennukleinsäuren. Bevorzugt ist daher der Fall, daß der Teil D2 der Amplifikatorsonde komplementär zu C2 oder einem Teil davon ist.

Die Trennung der Hybride aus Matrizennukleinsäure C und Verlängerungsprodukt des Spaltprodukts B' sowie gegebenenfalls der Matrizennukleinsäure C oder E und dem Verlängerungsprodukt des Spaltprodukts D' sowie eventueller weiterer Hybride (z. B. bei Verwendung weiterer Matrizennukleinsäuren) kann beispielsweise durch Hitzedenaturierung geschehen. Prinzipiell sind jedoch auch nicht-thermische Denaturierungsverfahren geeignet.

Die Verlängerung ungespaltener Amplifikatorsonden z. B. an den Verlängerungsprodukten kann analog zur Sondennukleinsäure B durch Blockierung des 3'-Endes der Amplifikatorsonden beispielsweise durch ein Didesoxynukleotid verhindert werden.

Die Temperatur des erfindungsgemäßen Verfahrens wird so gewählt, daß die Aktivitäten der eingesetzten Enzyme möglichst optimal sind und gleichzeitig die Hybridisierungsbedingungen ausreichende Spezifität erlauben. Bei der Verwendung nicht-thermostabiler RNAse ist beispielsweise ein Temperaturbereich zwischen 30° und 60°, besonders bevorzugt 42° zweckmäßig. Bei Einsatz einer thermostabilen RNAse sind höhere Temperaturen möglich. Für die Verlängerungsreaktion richtet sich die Temperatur ebenfalls nach dem für die Verlängerung eingesetzten Enzym. Dabei ist bei der Verwendung von thermostabilen Enzymen, z. B. Taq-DNA-Polymerase ein Bereich zwischen 50° und 80° bevorzugt und besonders bevorzugt ca. 60-70°. Durch Erhöhung der Aufenthaltswahrscheinlichkeit der Enzyme an den Reaktionsorten dürfte es möglich sein, die Umsatzraten zu steigern und somit die erforderliche Reaktionszeit zu senken oder die Sensitivität zu erhöhen.

Die Matrizennukleinsäure kann auch ein circuläres Molekül darstellen. Für diesen Fall ist die Verwendung eines Verlängerungsenzyms mit Strangverdrängungsaktivität bevorzugt.

Die Sondennukleinsäure, die Matrizennukleinsäuren und die Amplifikatomukleinsäuren können nach prinzipiell bekannten Verfahren hergestellt werden, sobald die Sequenz ihrer Teile festgelegt ist. Für den bevorzugten Fall, daß es sich um Oligonukleotide mit einer Länge von weniger als 100 Mononukleotidbausteinen handelt, ist die Synthese nach geläufigen chemischen Verfahren (z. B. Festphasensynthese analog Merrifield) bevorzugt. Dies ermöglicht auch die einfache Synthese gemischter Oligonukleotide (RNA/DNA-Chimäre). Für größere Nukleinsäuren sind rekombinante Herstellverfahren oder chemisch/enzymatische Verfahren, wie in EP-A-325970 beschrieben, bevorzugt. Teil B1 ist bevorzugt 12-35, Teil B2 bevorzugt 15-50, Teil C1 bevorzugt 10-100 und Teil C2 15-50 Nukleotide lang. Dasselbe gilt für die übrigen Matrizennukleinsäuren sowie die Amplifikatorsonden.

Figur 1 zeigt eine Grundausführung des erfindungsgemäßen Verfahrens, bei dem der Teil D2 der Amplifikatorsonde komplementär zu Teil C2 der Matrizennukleinsäure ist, und somit das Spaltprodukt D' an der Matrizennukleinsäure C verlängert werden kann.

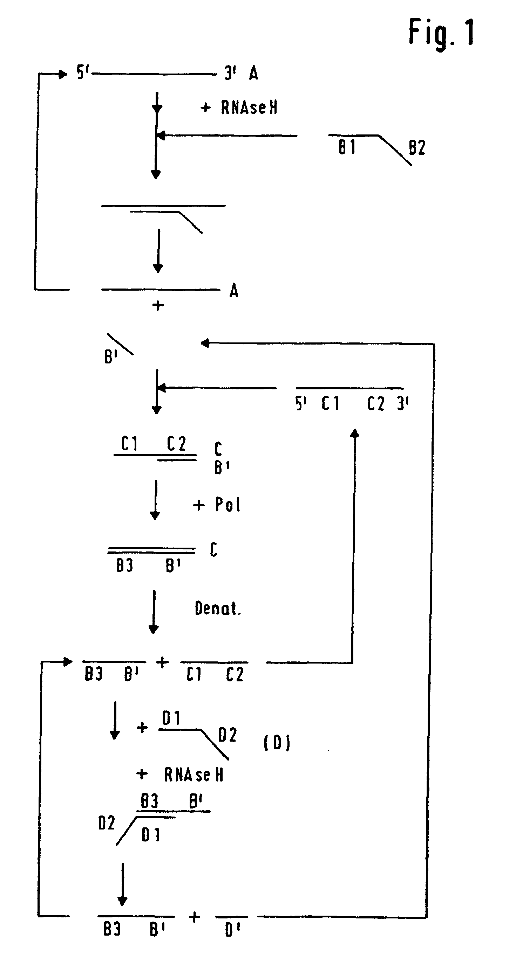

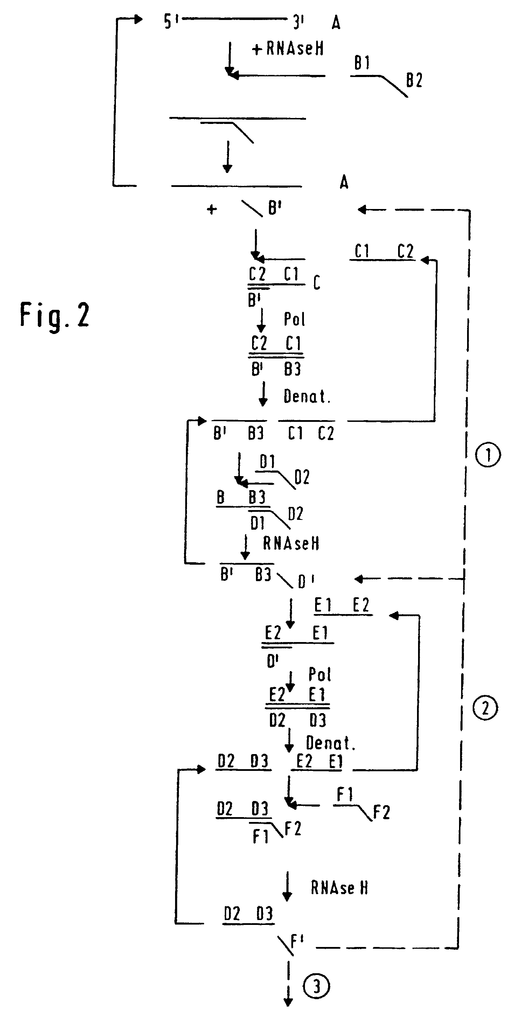

Figur 2 zeigt schematisch eine Ausführungsform, bei der das Spaltprodukt D' mit einer neuen Matrizennukleinsäure E hybridisiert wird. Durch Verwendung einer Sondennukleinsäure F kann ein Spaltstück F erzeugt werden, welches entweder so gewählt wird, daß es mit der Matrizennukleinsäure C (Weg 1), mit der Matrizennukleinsäure E (Weg 2) oder einer weiteren Matrizennukleinsäure G (Weg 3) hybridisieren kann.

Figur 3 und 4 zeigen Gele mit den Zwischenprodukten der Reaktion.

Figur 5 zeigt die Amplifikationsprodukte im Gel.

Die in den Figuren gezeigten Schritte können nacheinander unter Zugabe der jeweils benötigten Reagenzien durchgeführt werden. Alle benötigten Komponenten können bei entsprechendem Design der Komponenten jedoch auch schon zu Beginn der Reaktion zugesetzt werden, so daß der Prozess simultan abläuft.

Sofern die Denaturierung der Matrizennukleinsäure von dem Verlängerungsprodukt thermisch geschieht, müssen alle nicht-thermostabilen Komponenten jeweils nach Denaturierung erneut zugegeben werden. Insofern ist die Verwendung thermostabiler Enzyme, z. B. Thermus-aquaticus-Polymerase und thermostabile RNase H vorteilhaft.

Der Nachweis der Zielnukleinsäure A kann aufgrund eines der in der Amplifikation gebildeten Produkte bzw. Zwischenprodukte geführt werden. Er findet bevorzugt über den Nachweis einer Markierung in mindestens einer der verwendeten Sondenoder Matrizennukleinsäuren oder der gebildeten Spalt- oder Verlängerungsprodukte statt. Bevorzugt ist der Fall, daß ein Matrizenoligonukleotid mit einem Spaltprodukt unter geeigneten Hybridisierungsbedingungen zusammen mit einer DNA-Polymerase und markierten Deoxyribonukleosidtriphosphaten umgesetzt wird. Der Einbau markierter Mononukleotide kann beispielsweise nach Abtrennung der Nukleinsäuren von nicht-umgesetzten markierten Deoxyribonukleosidtriphosphaten nachgewiesen werden. Die Verwendung zweier unterschiedlich markierter Nukleotide ermöglicht den direkten festphasengebundenen Nachweis.

Die hohe Flexibilität und mögliche Doppelmarkierung, d. h. den während der Verlängerungsreaktion gleichzeitigen Einbau unterschiedlich markierter Desoxynukleotide ermöglichen die Anwendung des Verfahrens auf Teststreifen oder in Mikrotiterplatten oder den Nachweis über Durchflußcytometrie oder Kapillarelektrophorese.

Wenn in der Probe keine Zielnukleinsäure enthalten ist, hybridisiert das Matrizenoligonukleotid nur mit dem chimären RNA-DNA-Sondenmolekül. Eine Verlängerung des Sondenmoleküls kann nicht stattfinden, da das 3'-Ende nicht mit dem Matrizenoligonukleotid hybridisiert.

Das erfindungsgemäße Verfahren stellt ein Nachweisverfahren zur Verfügung, bei dem sowohl das Verlängerungsprodukt als auch die erzeugten Primer erneut in den Amplifikationszyklus zurückgeführt werden können. Als Amplifiationszyklus wird eine Reaktionsfolge verstanden, bei der ein Produkt einer Reaktion wiederum zur Erzeugung eines oder mehrerer dieser Produkte verwendet wird. In den Zeichnungen sind solche Zyklen durch einen Pfeil gekennzeichnet, der auf eine frühere Reaktionsstufe zurückführt. Dies bewirkt eine Amplifikation mit einem theoretischen Amplifikationsfaktor von mehr als 2^{x}, wenn x die Anzahl der Zyklen ist. Dahingehend ist also das erfindungsgemäße Verfahren den auf der PCR basierenden Verfahren überlegen. Außerdem kann es durch geeignetes Design zu noch höheren Amplifikationsraten ausgebaut werden.

Gegenstand der Erfindung ist außerdem ein Verfahren zur Erzeugung von Nukleinsäuresequenzen, enthaltend die Schritte
a) Verlängerung einer ersten Nukleinsäuresequenz um einen Verlängerungsbereich,
b) Hybridisierung einer zweiten Nukleinsäuresequenz, die länger als die erste Nukleinsäuresequenz ist und eine Sequenz enthält, die homolog zur ersten Nukleinsäuresequenz ist, mit dem Verlängerungsprodukt im Verlängerungsbereich,
c) Abbau der zweitenNukleinsäuresequenz um einen Teil oder die Gesamtheit der hybridisierten Sequenz,
d) Wiederholung der Schritte a) - c) mit den Abbauprodukten aus Schritt c).

Hierbei stellen die ersten Nukleinsäuresequenzen bevorzugt die oben genannten Primer bzw. Spaltprodukte dar. Die längere Nukleinsäuresequenz ist bevorzugt eine Sondennukleinsäure bzw. Amplifikatornukleinsäure. Der Verlängerungsbereich ist somit bevorzugt der Bereich D3. Der Abbau der längeren Nukleinsäure entspricht bevorzugt der Spaltung der Sondennukleinsäure bzw. der Amplifikatorsonde im Teil B1 bzw. D1 oder F1. Die Wiederholung der Schritte a) bis c) entspricht bevorzugt der Zyklusführung der Spaltprodukte.

Ein bevorzugter Gegenstand der Erfindung ist ein Verfahren zum empfindlichen Nachweis einer Zielnukleinsäure A durch
a) Hybridisierung der Zielnukleinsäure A mit einer Sondennukleinsäure B, die einen mit der Zielnukleinsäure hybridisierenden Teil B1 und einen nukleinsäurespezifischen Teil B2 enthält,
b) Spaltung der Sondennukleinsäure B im Teil B1,
c) Hybridisierung eines Spaltproduktes B' der Sondennukleinsäure, welches den nicht mit der Zielnukleinsäure hybridisierenden Teil B2 enthält, mit einer Matrizennukleinsäure C, die einen mit dem Spaltprodukt B' im Teil B2 hybridisierenden Teil C2 und einen nicht mit dem Teil B1 der Sondennukleinsäure hybridisierenden Teil C1 enthält,
d) Verlängerung des Spaltproduktes B' um einen zu Teil C1 ganz oder teilweise komplementären Nukleinsäureteil B3 und
e) Hybridisieren einer Amplifikatorsonde D mit dem Verlängerungsprodukt des Spaltprodukts B' in Teil B3, wobei die Sonde D einen Teil D1 enthält, der homolog zu C1 oder einem Teil davon ist und der spaltbar ist.

Für das oben genannte direkte Verfahren wird die Amplifikatorsonde D im Teil D1 gespalten, wobei ein Spaltprodukt D' entsteht. Dieses enthält beim direkten Verfahren eine Sequenz, die die Hybridisierung an die Matrizennukleinsäure C und die Verlängerung analog zur Verlängerung von B' ermöglicht. Hiermit wäre daher der Amplifikationszyklus geschlossen. Für das direkte Verfahren enthält daher die Amplifikatorsonde D zusätzlich zu Teil D1 einen nukleinsäurespezifischen Teil, welcher homolog zu B2 oder einem Teil davon ist.

Hierbei ist besonders bevorzugt, wenn der nukleinsäurespezifische Teil B2 möglichst nicht mit der Zielnukleinsäure hybridisiert. Bevorzugt findet zwischen Schritt d) und e) die Denaturierung des Hybrids aus dem Verlängerungsprodukt des Spaltprodukts B' mit der Matrizennukleinsäure C statt.

Besonders bevorzugt enthält das Verfahren im Fall des oben genannten indirekten Verfahrens außerdem die weiteren Schritte
f) Spaltung der Amplifikatorsonde D in Teil D1, vorzugsweise mit RNAse H,
   g) Hybridisierung des durch Spaltung erzeugten Spaltprodukt D' mit einer Matrizennukleinsäure E, die einen mit dem Spaltprodukt im Teil D2 hybridisierenden Teil E2 und einen nicht mit dem Teil D1 hybridisierenden Teil E1 enthält,
   h) Verlängerung des Spaltproduktes D' um einen zu Teil E1 komplementären Nukleinsäureteil D3 und
      optionell
   i) Hybridisieren einer Amplifikatorsonde F mit dem Verlängerungsprodukt des Spaltproduktes D' in Teil D3, wobei die Sonde F einen Teil F1 enthält, wobei F1 homolog zu C1 oder einem Teil davon oder homolog zu E1 oder einem Teil davon ist und wobei F1 spaltbar ist.

Für diesen Fall wird die Amplifikatorsonde D so gewählt, daß sie zusätzlich zu Teil D1 einen nukleinsäurespezifischen Teil D2 enthält, der jedoch nicht homolog zu B2 oder einem Teil davon ist. Besonders bevorzugt ist der Teil D2 spezifisch für den Teil E2 der Matrizennukleinsäure E.

Außerdem ist Gegenstand der Erfindung ein Set von Nukleinsäuren zum empfindlichen Nachweis von Zielnukleinsäuren enthaltend
a) eine Nukleinsäure B mit Teilen B1 und B2, wobei B1 einen zu einem Teil der Zielnukleinsäure komplementären und abbaubaren Teil und B2 einen nicht mit der Zielnukleinsäure hybridisierenden Teil darstellt,
b) eine Nukleinsäure C mit Teilen C1 und C2, wobei C1 einen weiteren nukleinsäurespezifischen Teil und C2 einen zu B2 komplementären Teil darstellt,
c) eine Nukleinsäure D mit einem Teil D1, wobei D1 homolog zu C1 oder einem Teil davon und spaltbar ist.

Bevorzugt enthält die Nukleinsäure D zusätzlich einen Teil D2, der homolog zu B2 oder einem Teil davon ist. In anderen Worten D2 komplementär zu C2.

Dies bedeutet, daß Teil D1 auch komplementär zu B3 ist.

Die erfindungsgemäßen Sets können außerdem die für den Nachweis erforderlichen Reagenzien insbesondere abbauende Enzyme, z. B. RNAse H, ein Nukleinsäureverlängerungsenzym, z. B. eine DNA-Polymerase oder reverse Trankriptase, Mononukleotide sowie für die Enzymreaktionen geeignet Puffer enthalten.

Das erfindungsgemäße Verfahren wird durch die folgenden Beispiele weiter erläutert:
Im Folgenden werden in Oligonukleotiden Großbuchstaben für Desoxyribonukleotideinheiten und Kleinbuchstaben für Ribonukleotideinheiten verwendet:

### Beispiel 1

### Vorgehensweise für die radioaktive Detektion:

1 pmol des RNA-DNA-Sondenmoleküles (B: 5'-GATCGGACTGGAAGTAATACGACTCACcgauacuaacauugagauucccg-3', SEQ.ID.NO. 1) werden mit jeweils unterschiedlichen Mengen der nachzuweisenden DNA (A: 5'-ATCTCGGGAATCTCAATGTTAGTATCGG-3', SEQ.ID.NO. 2) in einem Volumen von 20 µl bei 42°C für 3 h in Puffer P2 (10 mM Hepes, 1 mM MgCl2, pH 8,0) unter Zusatz von 3 µg RSA, 20 U RNasin und 4 U RNase H inkubiert. Dann werden die Ansätze mit je 1 pmol des Matrizenoligonukleotides (C: 5'-CGACGCCGCGTCGCAGAAGATCGGTGAGTCGTATTACTTCCAGTCCGATC -3', SEQ.ID.NO. 3) versetzt, für 1 min auf 100°C erhitzt und sofort in Eis abgekühlt. Anschließend wird jedem Ansatz 3 µl 10 x Taq-Puffer (100 mM Tris pH 8,3, 500 mM KCl, 15 mM MgCl₂, 1 mg/ml Gelatine), dNTPs (Endkonzentration je 1 mM dATP, dCTP, dGTP, dTTP) und 1 U Taq-Polymerase zugesetzt, die Mischungen auf 30 µl aufgefüllt und bei 60°C für 30 min inkubiert. Den Ansätzen werden je 10 pmol der 5'-terminal mittels gamma-[32P]-ATP und Polynukleotidkinase radioaktiv markierten Amplifikatorsonde zugefügt (D: 5'-GATCGGACTGGAAGTAATACGACTCACcgccgcgucgcagaagauc-3', SEQ.ID.NO. 4). (*) Nach erneuter Denaturierung (1 min, 100°C) werden jedem Ansatz 4 U RNase H zugegeben und bei 42°C für 3 h inkubiert. Es schließt sich ein weiterer Inkubationsschritt bei 60°C für 30 min an. Diese RNase H-katalysierte Degradations- und Taq-DNA-Polymerase-katalysierte Elongationsreaktion werden noch zweimal (ab *) wiederholt.

Für den Nachweis werden die Oligonukleotide (darunter die akkumulierten, 5'terminal radioaktiv markierten Elongationsprodukte des Spaltproduktes D' der Amplifikatorsonde) auf ein 12%-Sequenzgel (Sambrook et al. (1989) Molecular Cloning, Cold Spring Harbour Laboratory Press, S.6.36 ff.) aufgetragen und elektrophoretisch aufgetrennt. Die Identifizierung der elongierten Primer D' und damit der Rückschluß auf die Anwesenheit der Zielsequenz A erfolgt mittels Autoradiographie.

### Beispiel 2

### Vorgehensweise für die nicht-radioaktive Detektion:

10 pmol des RNA-DNA-Sondenmoleküles (B: 5'-GATCGGACTGGAAGTAATACGACTCACcgauacuaacauugagauucccg-3', SEQ.ID.NO. 1) werden mit jeweils unterschiedlichen Mengen der nachzuweisenden DNA (A: 5'-ATCTCGGGAATCTCAATGTTAGTATCGG-3', SEQ.ID.NO. 2) in einem Volumen von 20 µl bei 42°C für 3 h in Puffer P2 (10 mM Hepes, 1 mM MgC12, pH 8,0) unter Zusatz von 3 µg RSA, 20 U RNasin und 4 U RNase H inkubiert. Dann werden die Ansätze mit je 10 pmol des Matrizenoligonukleotides (C: 5'-CGACGCCGCGTCGCAGAAGATCGGTGAGTCGTATTACTTCCAGTCCGATC -3', SEQ.ID.NO. 3) versetzt, für 1 min auf 100°C erhitzt und sofort in Eis abgekühlt. Anschließend wird jedem Ansatz 3 µl 10 x Taq-Puffer (100 mM Tris pH 8,3, 500 mM KCl, 15 mM MgCl₂, 1 mg/ml Gelatine), dNTPs (Endkonzentration je 1 mM dATP, dCTP, dGTP, DIG-dUTP, BIO-dUTP) und 1 U Taq-Polymerase zugesetzt, die Mischungen auf 30 µl aufgefüllt und bei 60°C für 30 min inkubiert. Den Ansätzen werden je 100 pmol der Amplifikatorsonde zugefügt (D: 5'-GATCGGACTGGAAGTAATACGACTCACcgccgcgucgcagaagauc-3', SEQ.ID.NO. 4). (*) Nach erneuter Denaturierung (1 min, 100°C) werden jedem Ansatz 4 U RNase H zugegeben und bei 42°C für 3 h inkubiert. Es schließt sich ein weiterer Inkubationsschritt bei 60°C für 30 min an. Diese RNase H-katalysierte Degradations- und Taq-DNA-Polymerase-katalysierte Elongationsreaktion werden noch zweimal (ab *) wiederholt.

Nun werden nicht eingebaute Nukleotide mittels Ethanolpräziptiation der Oligonukleotide abgetrennt.

Für den Nachweis werden die gefällten Oligonukleotide (darunter die akkumulierten BIO-DIG-markierten Elongationsprodukte) in je 210 µl Puffer D (10 mM Hepes pH 8,3, 30 mM NaCl, 1 mM MnCl₂) aufgenommen. Die doppelt-markierten Syntheseprodukte werden in einer Streptavidin-beschichteten Mikrotiterplatte (MTP) immobilisiert (Streptavidin-beschichtete Mikrotiterplatte aus Reverse Transcriptase Assay, non-radioactive, Boehringer Mannheim 1468 120), indem je zwei mal 100 µl der Oligonukleotid-Lösungen in die Vertiefungen einer mit Waschpuffer (0,5% (V/V) Tween 20 in PBS ("phosphate buffered saline")) vorgewaschenen MTP pipettiert wurden. Die MTP wird unter Schütteln (Well-Warm1, Fa. Denley Instuments GmbH) für 1 h bei 37°C inkubiert.

Die MTP mit den immobilisierten Nukleinsäuremolekülen wird fünf mal mit je 200 µl Waschpuffer gewaschen. Dann werden je 100 µl der Konjugatverdünnung (polyklonales <DIG>-S-Fab-PODpoly-Konjugat (Boehringer Mannheim GmbH), 200 mU/ml in Konjugatpuffer (100 mM Na-Phosphat pH 7,5, 0,9% (G/V) NaCl, 1% (G/V) Ralufon F4J oder RSA Fraktion V; der Konjugatpuffer wird mit Diethylpyrocarbonat behandelt, sterilfiltriert (0,2 µm-Filter, Nalgene) und bei 4°C aufbewahrt)) zugegeben und die MTP erneut unter denselben Bedingungen inkubiert.

Ungebundene Konjugatmoleküle werden durch fünfmaliges Waschen entfernt. Nun werden pro Vertiefung 100 µl der Substratlösung (2,2'-azino-di-[3-ethylbenzthiezolinsulfonat(6)], ABTS) zugegeben. Die Farbreaktion erfolgt bei 37°C unter Schütteln. Die "Optische Dichte" des umgesetzten ABTS bei 405 nm wird nach kurzem Schütteln der MTP unmittelbar vor der Messung mittels eines ELISA-Readers (SLT) gegen den Referenzfilter von 492 nm gemessen und nach Abzug des Nullwertes (nur ABTS) die Mittelwerte der Doppelbestimmungen ermittelt (SLT Easy-Base Version 4.01).

### Beispiel 3

### Versuchsablauf mit Einbau von Digoxigenin

50 pmol Ziel-DNA A (5'-ATCTCGGGAATCTCAATGTTAGTATCGG-3', 28mer, SEQ.ID.NO. 2) wurden mit 50 pmol RNA-DNA-Sondenmolekül B (5'-GATCGGACTGGAAGTAATACGACTCACcgauacuaacauugagauucccg-3', 50mer, DNA-RNA-Oligo mit 23 Ribos am 3'-Ende, SEQ.ID.NO. 1) und 4 µl 10 x Puffer (100 mM Tris-HCl, 50 mM Kcl, 15 mM MgCl₂, pH 8,3) in einem Volumen von 40 µl unter Zusatz von 6 µg RSA, 40 U Rnasin und 4 U Rnase H 3 Std. bei 42°C inkubiert.

Nach der Inkubation wurden 20 µl zur Gelanalyse entnommen und mit 2 µl Auftragspuffer (6 x TBE, 30 % Glycerin, Bromphenolblau) gestoppt.

Für die "Auffüllreaktion" wurden zu den restlichen 20 µl des RNase H-Verdaus 25 pmol Matrizenoligo C (5'-CGACGCCGCGTCGCAGAAGATCGGTGAGTCGTATTACTTCCAGTCCGATC -3', 50mer, SEQ.ID.NO. 3) zugegeben, 1 min. bei 95°C denaturiert und sofort in Eis abgekühlt. Nach Zugabe von 0,5 µl 10 x Puffer (s.o.), je 100 µM dATP, dCTP und dGTP, 65 µM dTTP, 35 µM DIG-dUTP (jeweils Endkonzentration) und 2,5 U Taq-DNA-Polymerase wurde der Ansatz auf 25 µl aufgefüllt. Nach einer Inkubation von 30 min. bei 60°C wurde mit 3 µl Auftragspuffer (s.o.) abgestoppt

Die Proben wurden vollständig auf 20%iges Polyacrylamidgel (Biometra-Minigelkammer) aufgetragen.

Laufpuffer= 1 x TBE, Laufzeit ca. 1 Std. bei 250 V.

Anschließend erfolgte eine Färbung in 1 x TBE + EtBr (1 µg/ml). Nach Ansicht unter UV wurde das Gel auf eine Nylonmembran geblottet und diese UV-fixiert.

Die Detektion erfolgte nach Vorschrift des DIG-Systems (Boehringer Mannheim GmbH).

Analog zu diesem Ansatz wurde ein Ansatz ohne DIG-dUTP (mit 100 µM dTTP) durchgeführt und die Proben auf ein zweites Gel aufgetragen. Aus diesem Gel wurde nach dem Lauf und der Färbung mit EtBr die Bande von Spur 6 ausgeschnitten und die DNA lt Maniatis ("crush and soak" Methode nach Maxam und Gilbert, 1977) eluiert (in 20 µl TE-Puffer).

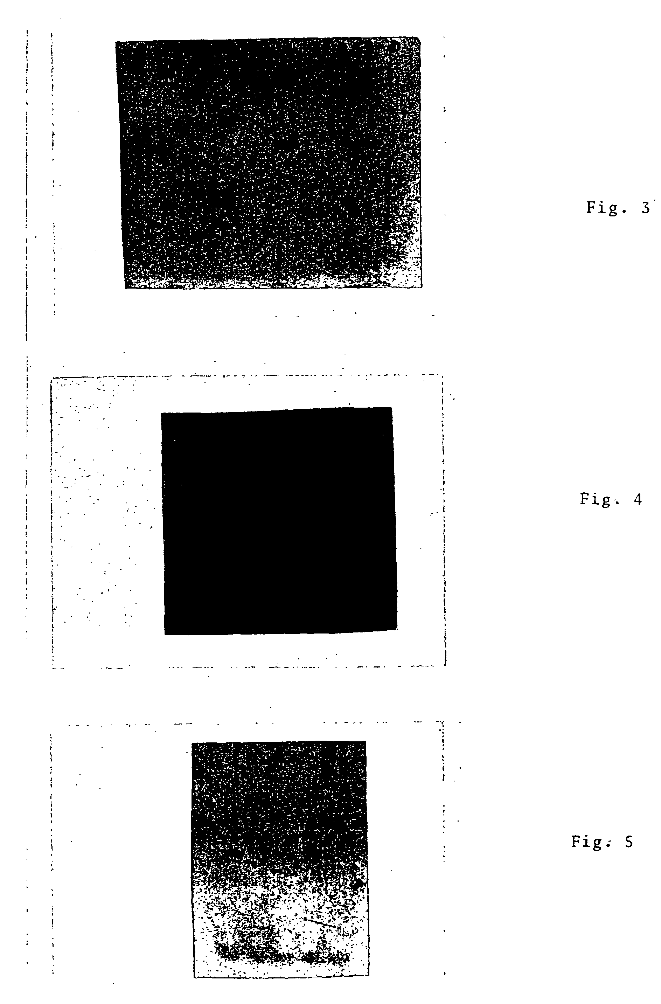

Figur 3 zeigt einzelne Stadien der Reaktion (Ethidiumbromidfärbung)

Figur 4 zeigt die Reaktion nach Blotten.

| | | | |
|---|---|---|---|
| Spur 1 | 25 pmol B | Spur 6 | wie 3 + 25pmol C → Auffüllreaktion |
| Spur 2 | 20 pmol C | Spur 7 | wie 4 → Auffüllreaktion |
| Spur 3 | 25pmol A + 25pmol B + RNase H | Spur 8 | wie 5 + 25pmol C → Auffüllreaktion |
| Spur 4 | 25pmol A + 25pmol B + RNase H | Spur 9 | 25pmol C → Auffüllreaktion |
| Spur 5 | 25pmol A + 25pmol B ohne Rnase H | Spur 10 | 25pmol C + 25pmol A → Auffüllreaktion |

### Versuchsablauf "Cyclus"

2 µl der eluierten DNA wurden mit 20 pmol Matrize C, 20 pmol Amplifikatorsonde (5'-GATCGGACTGGAAGTAATACGACTCAGcgccgcgucgcagaagauc-3', 46mer, DNA-RNA-Oligo mit 19 Ribos am 3'-Ende, SEQ.ID.NO. 4), 5 µl 10 x Puffer (s.o.), je 100 µM dATP, dCTP und dGTP, 65 µM dTTP, 35 µM DIG-dUTP (jeweils Endkonzentration), 40 U RNasin, 5 U thermostabiler RNase H und 2, 5 U Taq-DNA-Polymerase in einem Ansatz auf 50 µl aufgefüllt. Nach einem 3minütigen Denaturierungsschritt wurde folgender Cyclus 10mal wiederholt: 20 min. 42°C - 10 min. 60°C - 1 min. 95°C.

Nach 1,5 und 10 Cyclen wurden je 15 µl zur Analyse entnommen und mit 2 µl Auftragspuffer (s.o.) abgestoppt.

Die Proben wurden vollständig auf ein 20 % Polyacrylamidgel aufgetragen, Laufpuffer= 1 x TBE, Laufzeit ca. 1 Std. bei 250 V.

Anschließend erfolgte ein Blot auf Nylonmembran. Nach UV-Fixierung wurde der Blot lt. Vorschrift des DIG-Systems detektiert (FIG. 5).

| | |
|---|---|
| Spur 1 | DIG-markiertes Oligo, 46mer |
| Spur 2 | 1 Cyclus |
| Spur 3 | 5 Cyclen |
| Spur 4 | 10 Cyclen |

### SEQUENZPROTOKOLL

(1) ALLGEMEINE ANGABEN:
   (i) ANMELDER:
      (A) NAME: Boehringer Mannheim GmbH
      (B) STRASSE: Sandhoferstr. 116
      (C) ORT: Mannheim
      (E) LAND: DE
      (F) POSTLEITZAHL: 68298
      (G) TELEFON: 0621 759 4348
      (H) TELEFAX: 0621 759 4457
   (ii) BEZEICHNUNG DER ERFINDUNG: Verfahren zum besonders sensitiven Nachweis von Nukleinsaeuren
   (iii) ANZAHL DER SEQUENZEN: 4
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 50 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (iii) HYPOTHETISCH: NEIN
   (ix) MERKMAL:
      (A) NAME/SCHLÜSSEL: misc_feature
      (B) LAGE:1..27
      (D) SONSTIGE ANGABEN:/note= "DNA"
   (ix) MERKMAL:
      (A) NAME/SCHLÜSSEL: misc_feature
      (B) LAGE:28..50
      (D) SONSTIGE ANGABEN:/note= "RNA"
   (xi) SEQUENZBESCHREIBUNG: SEQ ID NO: 1:
(2) ANGABEN ZU SEQ ID NO: 2:
   (i) SEQUENZKENNZEICHEN:
      (A) LÄNGE: 28 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligodeoxyribonukleotid"
   (iii) HYPOTHETISCH: NEIN
   (xi) SEQUENZBESCHREIBUNG: SEQ ID NO: 2:
   ATCTCGGGAA TCTCAATGTT AGTATCGG 28
(2) ANGABEN ZU SEQ ID NO: 3:
   (i) SEQUENZKENNZEICHEN:
      (A) LÄNGE: 50 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligodeoxyribonukleotid"
   (iii) HYPOTHETISCH: NEIN
   (xi) SEQUENZBESCHREIBUNG: SEQ ID NO: 3:
(2) ANGABEN ZU SEQ ID NO: 4:
   (i) SEQUENZKENNZEICHEN:
      (A) LÄNGE: 46 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligoribonukleotid"
   (iii) HYPOTHETISCH: NEIN
   (ix) MERKMAL:
      (A) NAME/SCHLÜSSEL: misc_feature
      (B) LAGE:1..27
      (D) SONSTIGE ANGABEN:/note= "DNA"
   (ix) MERKMAL:
      (A) NAME/SCHLÜSSEL: misc_feature
      (B) LAGE:28..46
      (D) SONSTIGE ANGABEN:/note= "RNA"
   (xi) SEQUENZBESCHREIBUNG: SEQ ID NO: 4:

## Patentansprüche

1. Verfahren zum Nachweis von Zielnukleinsäuren, enthaltend die Schritte
a) zielsequenzabhängige Erzeugung einer Vielzahl von Nukleinsäuren, welche als Primer an einer Matrizennukleinsäure fungieren können, aus Nukleinsäuren mit einer längeren Sequenz,
b) Amplifikation der Nukleinsäuren, welche als Primer an einer Matrizennukleinsäure fungieren können, oder von Teilen davon,
c) Nachweis der Zielnukleinsäure aufgrund eines der in der Amplifikation gebildeten Produkte oder Zwischenprodukte.

2. Verfahren gemäß Anspruch 1, **dadurch gekennzeichnet, daß** die Erzeugung einer Vielzahl von Nukleinsäuren, welche als Primer an einer Matrizennukleinsäure fungieren können, durch Spaltung von Nukleinsäuren geschieht, die zwei Teile B 1 und B2 und Nukleinsäuren, welche als Primer an einer Matrizennukleinsäure fungieren können, beinhalten, wobei Teil B1 mit der Zielnukleinsäure oder einem Teil davon hybridisieren kann und gespalten werden kann, wenn er in mit der Zielnukleinsäure hybridisierter Form vorliegt, und den mit der Zielnukleinsäure nicht hybridisierenden Teil B2, der nicht gespalten werden kann, wenn Teil B1 in mit der Zielnukleinsäure hybridisierter Form vorliegt.

3. Verfahren gemäß Anspruch 1, **dadurch gekennzeichnet, daß** die Amplifikation der Nukleinsäuren, welche als Primer an einer Matrizennukleinsäure fungieren können geschieht durch Hybridisierung des Primers mit einer Matrizennukleinsäure, Verlängerung der Nukleinsäuren, welche als Primer an einer Matrizennukleinsäure fungieren können unter Verwendung der Matrizennukleinsäure als Templat, Hybridisierung des Verlängerungsprodukts mit einer Nukleinsäure, die einen nicht spaltbaren Teil D1 enthält, der auf seine gesamte Länge oder teilweise komplementär zu dem durch Verlängerung entstandenen Verlängerungsprodukts der Nukleinsäure, welche als Primer an einer Matrizennukleinsäure fungieren kann, ist und einen nicht mit der Zielnukleinsäure hybridisierenden Teil D2 enthält, und Spaltung der Nukleinsäure, die einen Teil D1 und D2 enthält, unter Freisetzung von Nukleinsäuren, welche als Primer an einer Matrizennukleinsäure fungieren können.

4. Verfahren gemäß Anspruch 1 zum empfindlichen Nachweis einer Zielnukleinsäure A durch
a) Hybridisierung der Zielnukleinsäure A mit einer Sondennukleinsäure B, die einen mit der Zielnukleinsäure hybridisierenden Teil B1 und einen nicht mit der Zielnukleinsäure hybridisierenden Teil B2 enthält,
b) Spaltung der Sondennukleinsäure B im Teil B1,
c) Hybridisierung eines Spaltproduktes B' der Sondennukleinsäure, welches den nicht mit der Zielnukleinsäure hybridisierenden Teil B2 enthält, mit einer Matrizennukleinsäure C, die einen mit dem Spaltprodukt B' im Teil B2 hybridisierenden Teil C2 und einen nicht mit dem Teil B1 der Sondennukleinsäure hybridisierenden Teil C1 enthält,
d) Verlängerung des Spaltproduktes B' um einen zu Teil C1 ganz oder teilweise komplementären Nukleinsäureteil B3 und
e) Hybridisieren einer Amplifikatorsonde D mit dem Verlängerungsprodukt des Spaltprodukts B' in Teil B3, wobei die Sonde D einen Teil D1 enthält, der homolog zu C1 oder einem Teil davon ist und der spaltbar ist,
f) Nachweis der Zielnukleinsäure A aufgrund eines der in den Schritten b) bis e) gebildeten Produkte.

5. Verfahren gemäß Anspruch 4, **dadurch gekennzeichnet, daß** die Spaltung der Sondennukleinsäure B mit Hilfe von RNAse H geschieht.

6. Verfahren gemäß Anspruch 4, **dadurch gekennzeichnet, daß** die Amplifikatorsonde D zusätzlich zu Teil D1 einen nicht mit der Zielnukleinsäure hybridisierenden Teil D2 enthält, welcher homolog zu B2 oder einem Teil davon ist.

7. Verfahren gemäß Anspruch 4, **gekennzeichnet, durch** die weiteren Schritte
f) Spaltung der Amplifikatorsonde D in Teil D1,
g) Hybridisierung des **durch** Spaltung erzeugten Spaltprodukt D' mit einer Matrizennukleinsäure E, die einen mit dem Spaltprodukt im Teil D2 hybridisierenden Teil E2 und einen nicht mit dem Teil D1 hybridisierenden Teil E1 enthält,
h) Verlängerung des Spaltproduktes D' um einen zu Teil E1 komplementären Nukleinsäureteil D3 und
i) optionell Hybridisieren einer Amplifikatorsonde F mit dem Verlängerungsprodukt des Spaltproduktes D' in Teil D3, wobei die Sonde F einen Teil F1 enthält, wobei F1 homolog zu C1 oder einem Teil davon oder homolog zu E1 oder einem Teil davon ist und wobei F1 spaltbar ist.

8. Verfahren gemäß Anspruch 4, **dadurch gekennzeichnet, daß** die Amplifikatorsonde D in Teil D1 mit RNAse H gespalten wird.

9. Verfahren zur Erzeugung von Nukleinsäuresequenzen, enthaltend die Schritte
a) Verlängerung einer ersten Nukleinsäuresequenz um einen Verlängerungsbereich,
b) Hybridisierung einer zweiten Nukleinsäuresequenz, die länger als die erste Nukleinsäuresequenz ist und eine Sequenz enthält, die homolog zur ersten Nukleinsäuresequenz ist, mit dem Verlängerungsprodukt im Verlängerungsbereich,
c) Abbau der zweitenNukleinsäuresequenz um einen Teil oder die Gesamtheit der hybridisierten Sequenz,
d) Wiederholung der Schritte a) - c) mit den Abbauprodukten aus Schritt c).

10. Kit zum empfindlichen Nachweis von Zielnukleinsäuren, enthaltend
a) eine Nukleinsäure B mit Teilen B1 und B2, wobei B1 einen zu einem Teil der Zielnukleinsäure komplementären und abbaubaren Teil und B2 einen nicht mit der Zielnukleinsäure hybridisierenden Teil darstellt,
b) eine Nukleinsäure C mit Teilen C1 und C2, wobei C1 einen weiteren nukleinsäurespezifischen Teil und C2 einen zu B2 komplementären Teil darstellt,
c) eine Nukleinsäure D mit einem Teil D1, wobei D1 homolog zu C1 oder einem Teil davon und spaltbar ist.

11. Kit gemäß Anspruch 10, **dadurch gekennzeichnet, daß** die Nukleinsäure D zusätzlich einen Teil D2 enthält, der homolog zu B2 bzw. komplementär zu C2 oder einem Teil davon ist.

## Claims

1. Method for detecting target nucleic acids comprising the following steps
a) target sequence-dependent generation of a multitude of nucleic acids which can act as primers on a template nucleic acid, from nucleic acids with a longer sequence,
b) amplification of the nucleic acids which can act as primers on a template nucleic acid, or of parts thereof,
c) detection of the target nucleic acid based on one of the products or intermediate products formed in the amplification.

2. Method as claimed in claim 1, **characterized in that** a multitude of nucleic acids which can act as primers on a template nucleic acid are generated by cleaving nucleic acids which contain two parts B1 and B2 and nucleic acids which can act as primers on a template nucleic acid where part B 1 can hybridize with the target nucleic acid or a part thereof and can be cleaved when it is present in a form in which it is hybridized with the target nucleic acid, and part B2 does not hybridize with the target nucleic acid and cannot be cleaved when part B1 is in a form in which it is hybridized with the target nucleic acid.

3. Method as claimed in claim 1, **characterized in that** the nucleic acids which can act as primers on a template nucleic acid are amplified by hybridizing the primer with a template nucleic acid, extending the nucleic acids which can act as primers on a template nucleic acid using the template nucleic acid as the template, hybridizing the extension product with a nucleic acid which contains a non-cleavable part D1 which is complementary over its entire length or partly complementary to the extension product formed by the extension of the nucleic acid which can act as a primer on a template nucleic acid and contains a part D2 which does not hybridize with the target nucleic acid, and cleaving the nucleic acid which contains a part D1 and D2 which releases nucleic acids which can act as primers on a template nucleic acid.

4. Method as claimed in claim 1 for the sensitive detection of a target nucleic acid A by
a) hybridizing the target nucleic acid with a probe nucleic acid B which contains a part B1 which hybridizes with the target nucleic acid and a part B2 which does not hybridize with the target nucleic acid,
b) cleaving the probe nucleic acid B in part B1,
c) hybridizing a cleavage product B' of the probe nucleic acid which contains the part B2 which does not hybridize with the target nucleic acid, with a template nucleic acid C which contains a part C2 which hybridizes with the cleavage product B' in part B2 and a part C1 which does not hybridize with part B1 of the probe nucleic acid,
d) extending the cleavage product B' by a nucleic acid part B3 which is completely or partly complementary to part C1 and
e) hybridizing an amplification probe D with the extension product of the cleavage product B' in part B3, said probe D containing a part D1 which is homologous to C1 or a part thereof and can be cleaved,
f) detecting the target nucleic acid A on the basis of one of the products formed in steps b) to e).

5. Method as claimed in claim 4, **characterized in that** the probe nucleic acid B is cleaved with the aid of RNAse H.

6. Method as claimed in claim 4, **characterized in that** the amplification probe D contains, in addition to part D1, a part D2 which does not hybridize with the target nucleic acid and is homologous to B2 or a part thereof.

7. Method as claimed in claim 4, **characterized by** the following additional steps
f) cleaving the amplification probe D in part D1,
g) hybridizing the cleavage product D' generated by the cleavage with a template nucleic acid E which contains a part E2 which hybridizes with the cleavage product in part D2 and a part E1 which does not hybridize with the part D1,
h) extending the cleavage product D' by a nucleic acid part D3 which is complementary to part E1 and
i) optionally hybridizing an amplification probe F with the extension product of the cleavage product D' in part D3, said probe F containing a part F1 which is homologous to C1 or a part thereof or is homologous to E1 or a part thereof and F1 can be cleaved.

8. Method as claimed in claim 4, **characterized in that** the amplification probe D is cleaved in part D1 by RNAse H.

9. Method for generating nucleic acid sequences comprising the following steps
a) extending a first nucleic acid sequence by an extension region,
b) hybridizing a second nucleic acid sequence which is longer than the first nucleic acid sequence and contains a sequence which is homologous to the first nucleic acid sequence, with the extension product in the extension region,
c) degrading the second nucleic acid sequence to remove a part or all of the hybridized sequence,
d) repeating steps a) - c) with the degradation products from step c).

10. Kit for the sensitive detection of target nucleic acids containing
a) a nucleic acid B comprising parts B1 and B2, where B1 is a degradable part which is complementary to a part of the target nucleic acid and B2 is a part which does not hybridize with the target nucleic acid,
b) a nucleic acid C comprising parts C1 and C2 where C1 is a further nucleic acid specific part and C2 is a part which is complementary to B2,
c) a nucleic acid D with a part D1 where D1 is homologous to C1 or a part thereof and can be cleaved.

11. Kit as claimed in claim 10, **characterized in that** the nucleic acid D additionally contains a part D2 which is homologous to B2 or is complementary to C2 or a part thereof.

## Revendications

1. Procédé pour le décèlement d'acides nucléiques cibles, contenant les étapes consistant à
a) générer, en fonction de la séquence cible, une multitude d'acides nucléiques qui peuvent faire office d'amorce sur un acide nucléique matriciel, à partir d'acides nucléiques possédant une séquence de longueur supérieure,
b) amplifier les acides nucléiques qui peuvent faire office d'amorce sur un acide nucléique matriciel, ou des parties des premiers cités,
c) déceler l'acide nucléique cible sur base d'un des produits ou des produits intermédiaires formés lors de l'amplification.

2. Procédé selon la revendication 1, **caractérisé en ce que** la génération d'une multitude d'acides nucléiques qui peuvent faire office d'amorce sur un acide nucléique matriciel a lieu par clivage d'acides nucléiques qui renferment deux parties B1 et B2 et des acides nucléiques qui peuvent faire office d'amorce sur un acide nucléique matriciel, la partie B1 pouvant s'hybrider avec l'acide nucléique cible ou avec une partie de ce dernier et pouvant s'en cliver lorsqu'elle est présente sous forme hybridée avec l'acide nucléique cible, et la partie B2 qui ne s'hybride pas avec l'acide nucléique cible, ne pouvant être séparée lorsque la partie B1 est présente sous forme hybridée avec l'acide nucléique cible.

3. Procédé selon la revendication 1, **caractérisé en ce que** l'amplification des acides nucléiques qui peuvent faire office d'amorce sur un acide nucléique matriciel a lieu par hybridation de l'amorce avec un acide nucléique matriciel, par prolongement des acides nucléiques qui peuvent faire office d'amorce sur un acide nucléique matriciel en utilisant l'acide nucléique matriciel comme matrice, par hybridation du produit de prolongement avec un acide nucléique qui contient une partie D1 inclivable qui manifeste une complémentarité partielle ou sur toute sa longueur par rapport au produit de prolongement de l'acide nucléique, obtenu via le prolongement, qui peut faire office d'amorce sur un acide nucléique matriciel, et qui contient une partie D2 qui ne s'hybride pas avec l'acide nucléique cible, et par clivage de l'acide nucléique qui contient une partie D1 et une partie D2, en libérant ainsi des acides nucléiques qui peuvent faire office d'amorce sur un acide nucléique matriciel.

4. Procédé selon la revendication 1, pour le décèlement sensible d'un acide nucléique cible A, par
a) hybridation de l'acide nucléique cible A avec un acide nucléique B faisant office de sonde qui contient une partie B1 qui s'hybride avec l'acide nucléique cible et une partie B2 qui ne s'hybride pas avec l'acide nucléique cible ;
b) clivage de l'acide nucléique B faisant office de sonde dans la partie B1 ;
c) hybridation d'un produit de clivage B' de l'acide nucléique faisant office de sonde, qui contient la partie B2 qui ne s'hybride pas avec l'acide nucléique cible, avec un acide nucléique matriciel C qui contient une partie C2 qui s'hybride avec le produit de clivage B' dans la partie B2 et une partie C1 qui ne s'hybride pas avec la partie B1 de l'acide nucléique faisant office de sonde ;
d) prolongement du produit de clivage B', le prolongement correspondant à une partie d'acides nucléiques B3 manifestant une complémentarité partielle ou totale par rapport à la partie C1 ; et
e) hybridation d'une sonde d'amplification D avec le produit de prolongement du produit de clivage B' dans la partie B3, la sonde D contenant une partie D1 qui manifeste une homologie par rapport à C1 ou par rapport à une partie de ce dernier et qui est clivable ;
f) décèlement de l'acide nucléique cible A sur base d'un des produits formés dans les étapes b) à e).

5. Procédé selon la revendication 4, **caractérisé en ce que** le clivage de l'acide nucléique B faisant office de sonde a lieu à l'aide de H-ribonucléase.

6. Procédé selon la revendication 4, **caractérisé en ce que** la sonde d'amplification D contient, en plus de la partie D1, une partie D2 qui ne s'hybride pas avec l'acide nucléique cible, manifestant une homologie par rapport à B2 ou par rapport à une partie de ce dernier.

7. Procédé selon la revendication 4, **caractérisé par** les étapes supplémentaires consistant à :
f) cliver la sonde d'amplification D dans la partie D1 ;
g) hybrider le produit de clivage D' obtenu par le clivage avec un acide nucléique matriciel E qui contient une partie E2 qui s'hybride avec le produit de clivage dans la partie D2 et une partie E1 qui ne s'hybride pas avec la partie D1 ;
h) prolonger le produit de clivage D', le prolongement correspondant à une partie d'acide nucléique D3 manifestant une complémentarité par rapport à la partie E1 ; et
i) le cas échéant, hybrider une sonde d'amplification F avec le produit de prolongement du produit de clivage D' dans la partie D3, la sonde F contenant une partie F1, ladite partie F1 manifestant une homologie par rapport à C1 ou par rapport à une partie de ce dernier ou une homologie par rapport à E1 ou par rapport à une partie de ce dernier et ladite partie F1 étant clivable.

8. Procédé selon la revendication 4, **caractérisé en ce que** la sonde d'amplification D est soumise à un clivage dans la partie D1 avec de la H-ribonucléase.

9. Procédé pour la génération de séquences d'acides nucléiques, contenant les étapes consistant à
a) prolonger d'une zone de prolongement une première séquence d'acides nucléiques ;
b) hybrider une deuxième séquence d'acides nucléiques, dont la longueur est supérieure à celle de la première séquence d'acides nucléiques et qui contient une séquence qui manifeste une homologie par rapport à la première séquence d'acides nucléiques, avec le produit de prolongement dans la zone de prolongement ;
c) soumettre à une dégradation la deuxième séquence d'acides nucléiques en supprimant une partie ou la totalité de la séquence hybridée;
d) répéter les étapes a) - c) avec les produits de dégradation de l'étape c).

10. Nécessaire pour le décèlement sensible d'acides nucléiques cibles, contenant
a) un acide nucléique B comprenant des parties B1 et B2, B1 représentant une partie dégradable et manifestant une complémentarité par rapport à une partie de l'acide nucléique cible et B2 représente une partie qui ne s'hybride pas avec l'acide nucléique cible ;
b) un acide nucléique C comprenant des parties C1 et C2, C1 représentant une partie supplémentaire spécifique à l'acide nucléique et C2 une partie manifestant une complémentarité par rapport à B2 ;
c) un acide nucléique D comprenant une partie D1, D1 manifestant une homologie par rapport à C1 ou par rapport à une partie de ce dernier et étant clivable.

11. Nécessaire selon la revendication 10, **caractérisé en ce que** l'acide nucléique D contient en outre une partie D2 manifestant une homologie par rapport à B2, respectivement une complémentarité par rapport à C2 ou par rapport à une partie de ce dernier.
